# EUROPEAN PATENT SPECIFICATION

(11) **EP 0 674 899 B1**
(45) Date of publication and mention of the grant of the patent: **22.08.2001**
(21) Application number: 95301809.0
(22) Date of filing: 17.03.1995
(51) Int. Cl.: A61K 7/32

(54) **Deodorant compositions**
Deodorant
Composition déodorant

(30) Priority: 31.03.1994 GB 9406554
(43) Date of publication of application: 04.10.1995
(73) Proprietor: UNILEVER PLC, London EC4P 4BQ (GB); UNILEVER N.V., 3013 AL Rotterdam (NL)
(72) Inventor: Correia, Martinho, Wetherby, West Yorkshire, LS22 5RB (GB); Timme, Susan Mary, Birstall, West Yorkshire, WF17 8BE (GB); Wight, Gordon Robert, Unilever Research, Wirral, Merseyside, L63 3JW (GB)
(74) Representative: Rots, Maria Johanna Francisca

(56) References cited:
- EP-A- 0 468 564
- EP-A- 0 684 038
- WO-A-92/00722
- GB-A- 2 091 553
- US-A- 5 082 652

## Description

This invention relates to aerosol deodorant products. In particular, it relates to concentrated deodorant products for topical application to the human skin.

The market in aerosol deodorant products is dominated by propellant driven aerosol products, which are often based on a relatively simple combination of deodorant agent (which in many cases is simply a short chain monohydric alcohol such as ethanol), together with a propellant and a perfume. Such compositions may typically additionally comprise various commonly used cosmetic adjuncts, such as skin feel improving agents, emollients, humectants, and antimicrobial compounds, to name but a few.

Such conventional compositions have many problems associated with them, of differing natures. To begin with, such compositions may have a tendency to produce a stinging sensation on or shortly after application, because of the relatively large amounts of alcohol that such compositions typically contain. Such a problem many be particularly acute with users who may have shaved the axillae shortly before application of the product.

A further problem that such compositions may face is related to environmental issues. There is increasing pressure throughout the world on products which use or dispense into the atmosphere relatively substantial quantities of either compounds which are believed to be deleterious to the atmosphere, in particular the ozone layer, or volatile organic compounds. The presence of such compounds in spray products is potentially under threat, and it is therefore desirable to minimise their presence in consumer products.

Weighed against this are the advantages that these compounds bring to consumer products, which is the reason why both propellants and volatile organic compounds have been used in these products for many years. In particular, the compounds involved (often short chain monohydric alcohols such as ethanol in deodorant compositions) are relatively cheap and efficacious.

It may also be considered desirable by some users to have a deodorant product packaged in as small a container as possible. This is so as to minimise the amount of materials used to make the product, but also relatively small products like this may have portability and convenience advantages to the user.

It is against this background that the invention sets out to improve on some or all of the disadvantages of the prior art. In particular, it is an object of the invention to provide aerosol deodorant products which have a similar or greater level of efficacy when compared to conventional deodorant packaged products, whilst at the same time using fewer of the compounds generally mentioned above, the use of which is recognised as being undesirable.

Thus, according to one aspect of the invention, there is provided a packaged concentrated deodorant composition for topical application to the human skin, comprising an aerosol container having a discharge valve, the valve being adapted to allow the contents of the container to be discharged at an initial discharge rate of less than 0.3 g/s, the composition comprising a perfume, a deodorant agent, a solvent vehicle for the composition, and a propellant.

Preferably, solvent for the composition may particularly act as a solvent vehicle for the deodorant agent, and may conveniently comprise a short chain (C₂-C₆) monohydric alcohol, such as ethanol or isopropanol. Ethanol in this instance is particularly preferred.

Preferably, the container for the packaged deodorant product has a filling volume of between 50-100 mls, more preferably 65-85 mls. Such a volume of container in conjunction with the packaged composition as described has been found to be particularly convenient, as it provides a container which is very readily portable. In the particular context of the invention, it also may provide a packaged deodorant product which may be substantially smaller than most commercially available products (which are typically two to three times this size), but which may last as long and prove at least as efficacious as these conventional products.

Preferably, the initial discharge rate (ie when the container is full) of the product is less than 0.30 g/s, and is more preferably between 0.3 and 0.1g/s.

The discharge valve in the container according to the invention will clearly be an important aspect of the hardware of the container. The provision of such a valve assembly to provide the required discharge rate will however be within the scope of the skilled man to determine, which will require no more than routine trial and error for optimisation. In this context, further information on how to combine the appropriate hardware may be found in for example US 5,068,099, or US 3,137,416, the contents of which are incorporated herein by reference.

Such compositions are advantageous over conventional compositions in that they have various associated environmental benefits, and also direct benefits for the user. In particular, packaged deodorant compositions according to the invention discharge less volatile organic compounds and propellant into the atmosphere with each usage, yet provide a product which has a comparable or even better efficacy compared to conventional deodorant products on the market.

An important feature of the invention is that, the efficacy of the product can be boosted by combining the vehicle of the composition (in particular when the vehicle is ethanol) with a deodorant active such as an inorganic salt, for example a metal salt, particularly zinc phenol sulphonate. Such a combination provides particularly good deodorancy in a product which has a relatively small size, and an initial discharge rate below 0.3 g/s. Satisfactory results may however be obtained with deodorant active salts of zinc, aluminium or zirconium.

In addition, because less product is being dispensed on each usage and it is discharged at a lower rate, there is a reduced tendency for the product to "bounce back" from the part of the body at which it is sprayed, hence providing less risk that the user will inhale the product. The lower discharge rate of such products also means that the product being dispensed has a lower impact velocity on the skin, which again leads to a reduced tendency of the product to bounce back. This also means that products may more readily be formulated which do not contain certain adhesive agents commonly found in such products, which are necessary to prevent excessive bounce back and therefore wastage of the product from the target surface. In this way the "respirable fraction" of the product is also reduced.

It has also been found that products according to the invention can be made relatively quiet in operation, thereby making them sensorially more acceptable to some users.

### The Deodorant Agent

The deodorant agent used in compositions according to the invention may be any deodorant agent which would readily be used by the skilled man in such compositions, but excluding short chain mono- and poly-hydric alcohols such as ethanol, isopropanol, and polyhydric alcohols such as propylene glycol.

Conveniently, the deodorant agent is solid, and may be an inorganic deodorant active. Preferred deodorant agents may typically be metal salts, such as those based on aluminium, zirconium, and zinc, or mixtures thereof. A particularly preferred deodorant agent is zinc phenolsulphonate. Preferably the deodorant agent is present in the composition at levels of from 1-6% by weight of the composition, more preferably 1.5-4% by weight of the composition.

### The Propellant

As an essential element of the invention, the packaged composition comprises a gaseous propellant to enable the composition to be dispensed from the container. Suitable propellants, which are normally liquefiable, include propane, n-butane, iso-butane and dimethyl ether, in particular dimethyl ether. The propellant gases may be used individually or in admixture, and with or without any other volatile liquids.

Examples of propellants which are not normally liquefiable under conditions of normal ambient use include carbon dioxide, nitrogen, nitrogen oxides and air, with these normally being used in a compressed state in conjunction with the suitable aerosol container.

Typically the propellant comprises 25-75% by weight of the packaged composition, more preferably 30-65% by weight, most preferably 30-60% by weight of the packaged composition.

### The Solvent Vehicle

The solvent vehicle in the packaged composition may comprise any cosmetically suitable liquid, but preferably comprises at least 20%, and preferably 30-60% of a short chain monohydric alcohol, in particular ethanol.

The composition may additionally comprise a polyhydric alcohol such as propylene glycol, dipropylene glycol and butylene glycol. Where a polyhydric alcohol is used in the composition, it is preferably used at a level of 0.2-5% by weight of the composition.

Other liquid vehicles for use in compositions according to the invention may include volatile silicones.

Preferably, the composition comprises no more than 10% water.

This has been found to be an important characteristic of certain compositions according to the invention since compositions which contain more than about 10% water tend to have poor sensory properties on application, feeling very wet on the users skin, and also taking a long time to dry. Compositions which contain too much water may also be incompatible with various commonly used propellants, and also certain preferred deodorant agents, such as zinc phenolsulphonate.

In particular, it is preferred that the solvent vehicle used in compositions according to the invention is capable of dissolving most of the solid components of the composition. the composition should preferably contain no particles greater than 75 µm. Any particles in the composition should preferably have a particle size in the region 10-75 µm, more preferably 55-75 µm.

### The Perfume

The composition according to the invention additionally comprises a perfume. This may be any conventional perfume such as perfume oil, but also includes so-called deoperfumes, which may provide their own deodorant effect, and are further described in EP 5,618, GB 2,016,507, EP 545,556 and GB 2,013,493 and EP 404,470, the contents of which are incorporated herein by reference.

Preferred perfumes for use in compositions according to the invention comprise compositions of perfume components which have a Deodorant Value of at least 0.25, preferably 0.5-3.0, as measured by the Deodorant Value Test described in GB 2013493, as well as satisfying the other criteria described therein.

Preferably, the perfume is present in the composition at a level of 0.01-2% by weight.

Compositions according to the invention may additionally comprise other cosmetics adjuncts typically found in deodorant compositions, including;
- non-volatile silicones, such as polydimethylsiloxan having a viscosity in excess of 5 mm²s⁻¹, for example from 50-1000 mm²s⁻¹, such as the Dow Corning 200 ™ fluids, and volatile silicones, such as linear or cyclic volatile silicones, such as Dow Corning fluids 344,345 or 200.
- emollients and humectants;
- preservatives and antioxidants.

The above list of cosmetic adjuncts is not considered exhaustive.

A particularly preferred aspect of the invention is a packaged composition which comprises a thickening agent. Such a thickened composition is advantageous because it overcomes other problems associated with conventional compositions. Notably, these problems relate to billowing and bounce back which can occur when conventional products are sprayed, and also dripping from the sprayed surface.

We have found that the use of a thickening agent in the composition to provide a composition having a viscosity of 10-100 mPas (cps) as measured with a Brookfield viscometer (disc spindle no.1) provides a composition which has a decreased tendency to cloud when is sprayed at a target surface. In addition, the thickening agent in the composition acts so as to minimise dripping or running of the composition when it is sprayed onto the skin.

In the above aspect of the invention, the preferred thickening agent used in compositions according to the invention is a synthetic cellulose derivative gum, in particular a hydroxypropyl cellulose derivative such as Klucel MF. Preferred hydroxy propyl cellulose derivatives have a relatively high molecular weight, for example over 2 x 10⁵; Klucel MF has a molecular weight of 6.5 x 10⁵. Preferably the thickening agent is used in compositions according to the invention at a level of 0.01-0.3% by weight of the composition, more preferably at a level of 0.05-0.1% by weight of the composition.

Such levels of thickening agent provide surprising benefits, in particular in aerosol forms of the invention. That is because it has been thought not possible previously to use hydroxypropyl cellulose thickeners in aerosol deodorant products, in particular those with a discharge rate less than 0.3 g/s, since it was previously thought that such use (in conjunction with the low flow rate) would lead to excessive levels of blockage in use.

The invention will now be described further by way of example only.

### Example 1

### Preparation

An aerosol product according to the invention can be prepared as follows. The preparation method is carried out at room temperature.

450.0 g of ethanol is used to dissolve 25.0 g of zinc phenol sulphonate (ex. Lohmann). To this is added 6.67 g of propyl glycol and 16.67 g of perfume. 0.625 g of hydroxypropyl cellulose (Klucel MFNF, ex Hercules Inc) is added to the base, with stirring, to thicken the base to a viscosity to 23.0 mPas (cps).

33.89 g of this base is then filled into a 75 ml aluminium can (ex Boxal). On to this is crimped a valve 22.5 g of dimethyl ether is then filled into the can by conventional techniques, and a dispensing button fitted. The final product contains 54.125% w/w ethanol, 3.00% w/w zinc phenol sulphonate, 0.80% w/w propylene glycol, 2.0% w/w perfume, 0.075% w/w Klucel ™, and 40% w/w dimethyl ether. The specific gravity of the composition is 0.753 g/cm³ at 20?C.

The preferred package for this package product had the following parameters.

The can is aluminium, ex Coster Tecnologie Speciali Spa, and preferably has a volume in the range 50-100 ml, preferably 75 ml with dimensions 35 x 126 mm. The package uses a spray through cap, the insert of which has a polyacetal insert with a terminal orifice of 0.45-0.55 mm. The valve used is the K123SL 90/3/4, ex Coster Tecnologie Speciali Spa, which has an aluminium cup/ferrule made of aluminium, with a size of 25.4 mm. The valve stem is made of polyacetyl, with an overall diameter of 3.90 mm and an orifice size of 0.4 mm. The valve housing is made of polyacetal, with an orifice (RTP) size of 0.4 mm. The diptube is a polypropylene capillary tube of internal diameter 1.25 mm.

For analogous compositions prepared generally according to the composition above, it was found that for compositions which were identical except that one contained no hydroxy propyl cellulose thickening agent, and the other containing 0.1% w/w hydroxy propyl cellulose, the respirable fraction of the spray in use was found to decrease by 40% for the one containing the hydroxyl propyl cellulose thickener, compared to the one without the thickener.

In addition, for analogous compositions prepared generally according to the composition above, it was found that for compositions which were identical except that one contained no hydroxy propyl cellulose thickening agent, and the other contained 0.25% w/w hydroxy propyl cellulose thickening agent, the capture efficiency of the spray in use was found to be double for the spray containing the thickening agent over the one containing no thickening agent.

## Claims

1. A packaged concentrated deodorant composition for topical application to the human skin, comprising an aerosol container having a discharge valve, the valve being adapted to allow the contents of the container to be discharged at an initial discharge rate of less than 0.3 g/s, the composition comprising a perfume, a deodorant agent, a solvent vehicle for the composition, and a propellant.

2. A packaged deodorant composition according to claim 1, additionally comprising a thickening agent.

3. A packaged deodorant composition according to claim 2, wherein the thickening agent is a hydroxypropyl cellulose derivative.

4. A packaged deodorant composition according to claim 3, wherein the hydroxypropyl cellulose derivative has a molecular weight of greater than 2 x 10⁵.

5. A packaged deodorant composition according to any of the preceding claims, wherein the solvent vehicle comprises a short chain monohydric alcohol.

6. A packaged deodorant composition according to any of the preceding claims, wherein the deodorant agent is a zinc, aluminium or zirconium salt.

7. A packaged deodorant composition according to any of the preceding claims, wherein the solvent vehicle additionally comprises 0.2-5% by weight of the total composition of a polyhydric alcohol.

8. A packaged deodorant composition according to any of the preceding claims, wherein the composition comprises less than 10% by weight of water.

9. A packaged deodorant composition according to any of the preceding claims, wherein the composition contains no particles having a particle size greater than 75µ.

## Patentansprüche

1. Eine verpackte konzentrierte Deodorant-Zusammensetzung für örtliche Anwendung auf die menschliche Haut, enthaltend einen Aerosol-Behälter mit einem Abflußventil, wobei das Ventil angepaßt ist, um es den Gehalten des Behälters zu erlauben, bei einer anfänglichen Ausflußmenge von weniger als 0,3 g/s ausgestoßen zu werden, die Zusammensetzung ein Parfüm, ein Deodorantmittel, einen Lösungsmittelträger für die Zusammensetzung und ein Treibmittel enthält.

2. Eine verpackte Deodorant-Zusammensetzung nach Anspruch 1, zusätzlich ein verdickendes Mittel enthaltend.

3. Eine verpackte Deodorant-Zusammensetzung nach Anspruch 2, worin das verdickende Mittel ein Hydroxypropylcellulose-Derivat ist.

4. Eine verpackte Deodorant-Zusammensetzung nach Anspruch 3, worin das Hydroxypropylcellulose-Derivat ein Molekulargewicht von größer als 2 × 10⁵ hat.

5. Eine verpackte Deodorant-Zusammensetzung nach irgendeinem der vorstehenden Ansprüche, worin der Lösungsmittelträger einen kurzkettigen einwertigen Alkohol enthält.

6. Eine verpackte Deodorant-Zusammensetzung nach irgendeinem der vorstehenden Ansprüche, worin das Deodorantmittel ein Zink-, Aluminium- oder Zirkonsalz ist.

7. Eine verpackte Deodorant-Zusammensetzung nach irgendeinem der vorstehenden Ansprüche, worin der Lösungsmittelträger zusätzlich 0,2 bis 5 Gew.-% der Gesamtzusammensetzung eines mehrwertigen Alkohols enthält.

8. Eine verpackte Deodorant-Zusammensetzung nach irgendeinem der vorstehenden Ansprüche, worin die Zusammensetzung weniger als 10 Gew.-% Wasser enthält.

9. Eine verpackte Deodorant-Zusammensetzung hach irgendeinem der vorstehenden Ansprüche, worin die Zusammensetzung keine Teilchen mit einer Teilchengröße von größer als 75 µm enthält.

## Revendications

1. Composition déodorante concentrée emballée pour application locale sur la peau humaine comprenant un récipient en aérosol ayant une soupape de décharge, la soupape étant adaptée pour permettre au contenu du récipient d'être déchargé à un taux de décharge initial de moins de 0,3 g/s, la composition comprenant un parfum, un agent déodorant, un véhicule solvant pour la composition et un propulseur.

2. Composition déodorante emballée selon la revendication 1, comprenant en plus un agent épaississant.

3. Composition déodorante emballée selon la revendication 2, dans laquelle l'agent épaississant est un dérivé d'hydroxypropylcellulose.

4. Composition déodorante selon la revendication 3, dans laquelle le dérivé d'hydroxypropylcellulose a une masse moléculaire de plus de 2 x 10⁵.

5. Composition déodorante emballée selon l'une quelconque des revendications précédentes, dans laquelle le véhicule solvant comprend un monoalcool à chaîne courte.

6. Composition déodorante emballée selon l'une quelconque des revendications précédentes, dans laquelle l'agent déodorant est un sel de zinc, aluminium ou zirconium.

7. Composition déodorante emballée selon l'une quelconque des revendications précédentes, dans laquelle le véhicule solvant comprend de plus 0,2 à 5% en poids de la composition totale d'un polyalcool.

8. Composition déodorante emballée selon l'une quelconque des revendications précédentes, dans laquelle la composition comprend moins de 10% en poids d'eau.

9. Composition déodorante emballée selon l'une quelconque des revendications précédentes, dans laquelle la composition ne contient pas de particules ayant une granulométrie supérieure à 75 *µ*m.
